# EUROPEAN PATENT APPLICATION

(11) **EP 4 640 710 A1**
(43) Date of publication of application: **29.10.2025**
(21) Application number: 23907877.7
(22) Date of filing: 22.12.2023
(51) Int. Cl.: C07K 16/28, A61P 35/00, A61K 39/39, A61K 47/68, G01N 33/574, A61K 39/00

(54) **MONOCLONAL ANTIBODY SPECIFICALLY BINDING TO CLAUDIN-18.2 AND USE THEREOF**

(30) Priority: 23.12.2022 KR 20220182621
(71) Applicant: Y-Biologics Inc., Daejeon 34013 (KR); Trioar, Inc., Daejeon, 34037 (KR)
(72) Inventor: PARK, Bum-Chan, Daejeon 34013 (KR); KIM, Soo Young, Daejeon 34013 (KR); PARK, Min Young, Daejeon 34013 (KR); SHIN, Ji-Young, Daejeon 34013 (KR); YU, Seon Mi, Daejeon 34013 (KR); PARK, Ok Ku, Daejeon 34037 (KR); LEE, Hyun Mi, Daejeon 34037 (KR); LEE, Hyun Ju, Daejeon 34037 (KR); PARK, Su Ho, Daejeon 34037 (KR); CHO, Jong Un, Daejeon 34037 (KR); WOO, Sung Ho, Daejeon 34037 (KR)
(74) Representative: Ipsilon
(86) International application number: PCT/KR2023/021443
(87) International publication number: WO 2024/136594

(57) **Abstract**

The present invention relates to a monoclonal antibody that specifically binds to claudin-18.2 (claudin-18 spliced variant 2, CLDN18.2) and use thereof. As the antibody of the present invention has high binding property for CLDN18.2 and excellent internalization capability, it can be advantageously employed in the development of cancer therapeutic agents in the form of monoclonal antibodies or modified formats (antibody-drug conjugates, chimeric antigen receptors, or multi-specific antibodies).

## Description

### Technical Field

The present invention relates to a monoclonal antibody specifically binding to claudin-18.2 (claudin-18 spliced variant 2, CLDN18.2), and use thereof.

### Background Art

Claudin-18 is a member of the endogenous membrane protein family located at the junctions of epithelial and endothelial cells, and has a molecular weight of approximately 27 kDa. Its structure consists of two extracellular loops and four transmembrane (TM) domains. There are two splice variants of claudin-18, which differ by only eight amino acids: claudin-18.1 (GenBank accession number NM_016369) and claudin-18.2 (GenBank accession number NM_001002026). Claudin-18.1 is expressed in normal lung and gastric epithelium, whereas claudin-18.2 expression is highly restricted in normal tissues except for the gastric mucosa. However, claudin-18.2 is overexpressed in various cancers and their metastatic forms, including gastric cancer, liver cancer, cholangiocarcinoma, breast cancer, kidney cancer, pancreatic cancer, non-small cell lung cancer, and mesothelioma (Okugawa T, et al., Dig Dis Sci (2012) 57: 1562-7; Rohde C, et al., Jpn J Clin Oncol (2019) 49: 870-6). Claudin proteins may play important roles in tumorigenesis, metastasis, and inflammation. It is known that altered expression of claudin proteins may lead to impaired tight junction function and affect signaling pathways that promote tumor progression.

Accordingly, in order to develop an effective anti-cancer therapeutic agent, it is necessary to develop an antibody that specifically binds to claudin-18.2, which, unlike claudin-18.1, exhibits tumor-specific expression.

Recently, a potent chimeric IgG1 monoclonal antibody (mAb) named zolbetuximab, which binds to claudin-18.2 on the surface of tumor cells, has been developed and is currently undergoing clinical trials. In particular, in a phase III clinical trial (SPOTLIGHT trial, NCT03504397), zolbetuximab, used as a first-line therapeutic agent, improved progression-free survival (PFS) and overall survival (OS) in patients with claudin-18.2-positive gastric cancer compared to standard chemotherapy.

In light of the above technical background, the inventors of the present invention used human claudin-18.2 protein as an antigen to screen for monoclonal antibodies that specifically bind to human claudin-18.2, using a fully human antibody sequence-based phage display technology. The monoclonal antibodies in human antibody form, generated based on the variable regions of the heavy and light chains of the screened antibody clones, were found to selectively bind to the human CLDN18.2 protein expressed on the surface of cells and exhibit excellent internalization property, thereby the present invention is completed.

Meanwhile, Korean Patent Publication No. 2022-0136267 discloses "Antibody-drug conjugate comprising an antibody against human CLDN18.2 and use thereof," and Korean Patent Publication No. 2022-0121873 discloses "Anti-claudin-18.2 antibody and use thereof." However, "Monoclonal antibody specifically binding to claudin-18.2 and use thereof" of the present invention is not disclosed before.

### DETAILED DESCRIPTION OF THE INVENTION

### Technical Problems to be Solved

The object of the present invention is to provide a novel antibody against claudin-18.2 (claudin-18 spliced variant 2, CLDN18.2) or an antigen-binding fragment thereof.

Another object of the present invention is to provide a polynucleotide encoding the antibody or an antigen-binding fragment thereof.

Another object of the present invention is to provide a vector comprising the polynucleotide, a transformant into which the vector has been introduced, and a method for producing the same.

Yet another object of the present invention is to provide a composition for cancer treatment comprising the antibody or an antigen-binding fragment thereof.

### Technical Means for Solving Problems

To solve the above problem, the present invention provides a monoclonal antibody that specifically binds to claudin-18.2 (claudin-18 spliced variant 2, CLDN18.2), comprising a heavy chain variable region containing heavy chain CDR (complementarity-determining region) 1 as described in SEQ ID NO: 1, heavy chain CDR2 as described in SEQ ID NO: 2, and heavy chain CDR3 as described in SEQ ID NO: 3, and a light chain variable region containing light chain CDR1 as described in SEQ ID NO: 5, light chain CDR2 consisting of the amino acid sequence DVS, and light chain CDR3 as described in SEQ ID NO: 6, or an antigen-binding fragment thereof.

The present invention further provides a monoclonal antibody that specifically binds to claudin-18.2, comprising a heavy chain variable region containing heavy chain CDR1 as described in SEQ ID NO: 1, heavy chain CDR2 as described in SEQ ID NO: 2, and heavy chain CDR3 as described in SEQ ID NO: 8, and a light chain variable region containing light chain CDR1 as described in SEQ ID NO: 10, light chain CDR2 consisting of the amino acid sequence AAS, and light chain CDR3 as described in SEQ ID NO: 11, or an antigen-binding fragment thereof.

The present invention further provides a monoclonal antibody that specifically binds to claudin-18.2, comprising a heavy chain variable region containing heavy chain CDR1 as described in SEQ ID NO: 1, heavy chain CDR2 as described in SEQ ID NO: 2, and heavy chain CDR3 as described in SEQ ID NO: 13, and a light chain variable region containing light chain CDR1 as described in SEQ ID NO: 15, light chain CDR2 consisting of the amino acid sequence GAF, and light chain CDR3 as described in SEQ ID NO: 11, or an antigen-binding fragment thereof.

The present invention further provides a polynucleotide encoding the heavy chain variable region and the light chain variable region of the monoclonal antibody or an antigen-binding fragment thereof.

The present invention further provides a recombinant expression vector comprising the polynucleotide.

The present invention further provides a non-human transformant transformed with the expression vector.

The present invention further provides a method for producing a monoclonal antibody that specifically binds to claudin-18.2 or an antigen-binding fragment thereof by culturing the transformant.

The present invention further provides a pharmaceutical composition for treating cancer comprising the monoclonal antibody or an antigen-binding fragment thereof as an active ingredient.

The present invention further provides a method for treating cancer, comprising administering the pharmaceutical composition for treating cancer to a subject in need thereof.

The present invention further provides a cancer diagnostic composition comprising the monoclonal antibody or an antigen-binding fragment thereof, and a cancer diagnostic kit comprising the cancer diagnostic composition.

The present invention further provides an antibody-drug conjugate comprising the monoclonal antibody or an antigen-binding fragment thereof conjugated with a drug.

The present invention further provides a chimeric antigen receptor (CAR) protein comprising: i) the monoclonal antibody; ii) a transmembrane domain; and iii) an intracellular signaling domain characterized in that it induces T cell activation upon binding of the antibody of i) to an antigen.

The present invention still further provides a multi-specific antibody comprising the monoclonal antibody or an antigen-binding fragment thereof.

### Advantageous Effect of the Invention

The novel monoclonal antibody that binds to claudin-18.2 (CLDN18.2) according to the present invention consists of a fully human antibody sequence capable of cellular internalization, exhibiting specific antigen-binding property and a variety of internalization properties. Given its confirmed thermal stability, the monoclonal antibody or its modified forms (such as antibody-drug conjugates, chimeric antigen receptors, or multi-specific antibodies) are expected to be advantageously used in the treatment and/or prevention of cancer diseases and related disorders that express claudin-18.2.

### Brief Description of the Drawings

Figure 1 is a flow cytometry analysis result using HEK293E cell line, which does not express human claudin-18.2 (CLDN18.2).
Figure 2 is a flow cytometry analysis result using HEK293E cell line overexpressing CLDN18.2 (HEK293E/18.2).
Figure 3 is a flow cytometry analysis result using CHO-K1 cell line overexpressing CLDN18.2 (CHO-K1/18.2).
Figure 4 is an ELISA analysis result using human CLDN18.2 as the coated antigen.
Figure 5 is an ELISA analysis result using human CLDN18.1 as the coated antigen.
Figure 6 is a flow cytometry analysis result using BxPC3 cell line (a cell line endogenously expressing human CLDN18.1).
Figure 7 is a result of flow cytometry analysis for determining the expression level of CLDN18.1 in the BxPC3 cell line used in Figure 6.
Figure 8 shows an analysis of the internalization property of the monoclonal antibody in HEK293E cell line (CLDN18.2 negative) using a live cell analysis system (IncuCyte).
Figure 9 shows an analysis of the internalization property of the monoclonal antibody in HEK293E/18.2 cell line (CLDN18.2 overexpressing) using a live cell analysis system (IncuCyte).
Figures 10 and 11 show analyses of the internalization property of the monoclonal antibody in CHO-K1/18.2 cell line (CLDN18.2 overexpressing) using cell immunofluorescence staining.
Figure 12 shows an analysis of the internalization and cytolytic activity of the monoclonal antibody in HEK293E/18.2 cell line (CLDN18.2 overexpressing) using a FabZAP assay.
Figure 13 shows an analysis of the internalization and cytolytic activity of the monoclonal antibody in HEK293E cell line (CLDN18.2 negative) using a FabZAP assay.
Figure 14 shows an analysis of the thermal stability of the CLDN18.2-specific monoclonal antibody using differential scanning fluorimetry (DSF).

### Best Mode(s) for Carrying out the Invention

To achieve the object of the present invention, the present invention provides a monoclonal antibody that specifically binds to claudin-18.2 (claudin-18 spliced variant 2, CLDN18.2), comprising a heavy chain variable region containing heavy chain CDR (complementarity-determining region) 1 as described in SEQ ID NO: 1, heavy chain CDR2 as described in SEQ ID NO: 2, and heavy chain CDR3 as described in SEQ ID NO: 3, and a light chain variable region containing light chain CDR1 as described in SEQ ID NO: 5, light chain CDR2 consisting of the amino acid sequence DVS, and light chain CDR3 as described in SEQ ID NO: 6, or an antigen-binding fragment thereof.

The present invention further provides a monoclonal antibody that specifically binds to claudin-18.2, comprising a heavy chain variable region containing heavy chain CDR1 as described in SEQ ID NO: 1, heavy chain CDR2 as described in SEQ ID NO: 2, and heavy chain CDR3 as described in SEQ ID NO: 8, and a light chain variable region containing light chain CDR1 as described in SEQ ID NO: 10, light chain CDR2 consisting of the amino acid sequence AAS, and light chain CDR3 as described in SEQ ID NO: 11, or an antigen-binding fragment thereof.

The present invention further provides a monoclonal antibody that specifically binds to claudin-18.2, comprising a heavy chain variable region containing heavy chain CDR1 as described in SEQ ID NO: 1, heavy chain CDR2 as described in SEQ ID NO: 2, and heavy chain CDR3 as described in SEQ ID NO: 13, and a light chain variable region containing light chain CDR1 as described in SEQ ID NO: 15, light chain CDR2 consisting of the amino acid sequence GAF, and light chain CDR3 as described in SEQ ID NO: 11, or an antigen-binding fragment thereof.

The monoclonal antibody that specifically binds to claudin-18.2 according to the present invention, comprising a heavy chain variable region containing heavy chain CDR1 as described in SEQ ID NO: 1, heavy chain CDR2 as described in SEQ ID NO: 2, and heavy chain CDR3 as described in SEQ ID NO: 3, and a light chain variable region containing light chain CDR1 as described in SEQ ID NO: 5, light chain CDR2 consisting of the amino acid sequence DVS, and light chain CDR3 as described in SEQ ID NO: 6, can be more specifically a monoclonal antibody that comprises a heavy chain variable region described by the amino acid sequence of SEQ ID NO: 4 and a light chain variable region described by the amino acid sequence of SEQ ID NO: 7,
the monoclonal antibody that specifically binds to claudin-18.2 comprising a heavy chain variable region containing heavy chain CDR1 as described in SEQ ID NO: 1, heavy chain CDR2 as described in SEQ ID NO: 2, and heavy chain CDR3 as described in SEQ ID NO: 8, and a light chain variable region containing light chain CDR1 as described in SEQ ID NO: 10, light chain CDR2 consisting of the amino acid sequence AAS, and light chain CDR3 as described in SEQ ID NO: 11, can be more specifically a monoclonal antibody that comprises a heavy chain variable region described by the amino acid sequence of SEQ ID NO: 9 and a light chain variable region described by the amino acid sequence of SEQ ID NO: 12, and
the monoclonal antibody that specifically binds to claudin-18.2 comprising a heavy chain variable region containing heavy chain CDR1 as described in SEQ ID NO: 1, heavy chain CDR2 as described in SEQ ID NO: 2, and heavy chain CDR3 as described in SEQ ID NO: 13, and a light chain variable region containing light chain CDR1 as described in SEQ ID NO: 15, light chain CDR2 consisting of the amino acid sequence GAF, and light chain CDR3 as described in SEQ ID NO: 11, can be more specifically a monoclonal antibody that comprises a heavy chain variable region described by the amino acid sequence of SEQ ID NO: 14 and a light chain variable region described by the amino acid sequence of SEQ ID NO: 16.

In the present invention, the term "monoclonal antibody" refers to a protein molecule that is directed against a single antigenic site (single epitope) and specifically binds thereto. The monoclonal antibody can be produced using various methods that are known in the pertinent art.

In the present invention, the term "antibody" refers to an antibody in whole antibody form, which has a structure consisting of two full-length light chains and two full-length heavy chains, with each light chain being linked to a heavy chain by a disulfide bond. The whole antibody includes IgA (immunoglobulin A), IgD, IgE, IgM, and IgG, with IgG having subtypes of IgG1, IgG2, IgG3, and IgG4. Additionally, the constant region of the heavy chain includes the types gamma (γ), mu (µ), alpha (α), delta (δ), and epsilon (ε), with subclasses of gamma1 (γ1), gamma2 (y2), gamma3 (γ3), gamma4 (γ4), alpha1 (α1), and alpha2 (α2). The constant region of the light chain includes the kappa (κ) and lambda (λ) types.

The monoclonal antibody of the present invention may be selected from the group consisting of a fully human antibody, a humanized antibody, a chimeric antibody, a mouse antibody, and a recombinant antibody. Preferably, it may be a fully human antibody, but is not limited thereto. Since the fully human antibody has a structure that is derived entirely from human sources, it has the advantage of a lower probability of inducing immune responses compared to conventional humanized or mouse antibodies, thus minimizing the risk of undesirable immune responses when administered to humans. Due to such advantage, it is highly useful as a therapeutic antibody.

In the present invention, the term "antigen-binding fragment" refers to a fragment that possesses antigen-binding functionality, including Fab, F(ab'), F(ab')₂, and Fv. Among antibody fragments, Fab has a structure that includes the variable regions of the light and heavy chains, the constant region of the light chain, and the first constant region (CH1) of the heavy chain, with one antigen-binding site. F(ab') differs from Fab in that it contains a hinge region at the C-terminal of the heavy chain's CH1 domain, which includes one or more cysteine residues. F(ab')₂ antibodies are generated when the cysteine residues in the hinge region of Fab' form a disulfide bond. Fv is the smallest antibody fragment, containing only the variable regions of the heavy and light chains. The recombinant technology for producing Fv fragments is disclosed in the International Publication No. WO 88/10649. The double-chain Fv (dsFv) connects the variable regions of the heavy and light chains through a disulfide bond, while the single-chain Fv (scFv) is typically connected via a peptide linker that covalently links the variable regions of the heavy and light chains. These antibody fragments can be obtained using proteolytic enzymes (e.g., papain cleavage of whole antibodies to obtain Fab, or pepsin cleavage to obtain F(ab')₂ fragments), but are preferably produced through recombinant DNA technology.

In the present invention, the term "variable region" refers to the region that performs the function of specifically binding to an antigen, and shows considerable sequence variability. The variable region contains complementarity-determining regions (CDRs) 1, 2, and 3 (i.e., CDR1, CDR2, and CDR3). Between these complementarity-determining regions, there is a framework region (FR) that supports the loops of the complementarity-determining regions.

In the present invention, the term "complementarity-determining region (CDR)" refers to a loop-shaped region involved in the recognition of an antigen, in which the sequence variation of this region determines the specificity of the antibody for an antigen.

According to one embodiment of the present invention, the inventors of the present invention screened for scFvs specifically binding to claudin-18.2 from a native human single chain Fv library, and then converted them into IgG form to produce human antibodies that specifically bind to claudin-18.2.

In the present invention, the term "biopanning" refers to a process of selecting phages that expresses a peptide on their surface, which possesses the property of binding to target molecules (such as antibodies, enzymes, cell surface receptors, etc.) from a phage library that displays peptides on the outer wall (coat) of the phage.

The monoclonal antibody according to the present invention or an antigen-binding fragment thereof is not particularly limited, but may be glycosylated and/or PEGylated to increase the retention time in the body after administration.

The term "glycosylation" used in the present invention refers to a processing method that transfers a glycosyl group to a protein. The glycosylation is performed by glycosyltransferases, in which the glycosyl group is bound to serine, threonine, asparagine, or hydroxylysine residues of a target protein. The glycosylated protein can be used not only as a constitutional component in biological tissues but also plays a crucial role in cellular recognition on the cell surface. Therefore, in the present invention, the glycosylation of the monoclonal antibody or an antigen-binding fragment thereof, or the modification of the glycosylation pattern, can be employed to enhance the efficacy of the antibody.

The term "PEGylation" used in the present invention refers to a processing method in which polyethylene glycol (PEG) is introduced to the monoclonal antibody or an antigen-binding fragment thereof to improve the antibody's retention time in blood. Specifically, PEGylation of polymeric nanoparticles with polyethylene glycol increases the hydrophilicity of the nanoparticle surface, preventing recognition by immune functions, including macrophages in human body, which phagocytose and digest pathogens, waste products, and foreign substances. This results in a so-called "stealth effect," preventing rapid degradation in the body. Consequently, PEGylation enhances the retention time of the antibody in blood. In the present invention, PEGylation may be achieved by the coupling of the carboxyl group of hyaluronic acid and the amine group of polyethylene glycol to form an amide group, but it is not limited thereto, and various methods of PEGylation may be employed. The polyethylene glycol used is not particularly limited, but preferably has a molecular weight between 100 and 1,000 and has a linear or branched structure.

The glycosylation and/or PEGylation can be modified with various patterns of glycosylation and/or PEGylation using methods that are known in the pertinent art, as long as the functionality of the antibody of the present invention is maintained. The antibody of the present invention includes the variant monoclonal antibody with variously modified glycosylation and/or PEGylation patterns, or an antigen-binding fragment thereof.

The term "affinity" refers to the ability to specifically recognize and bind to a particular region of the antigen. High affinity, along with the specificity of the antibody for the antigen, is an important factor in the immune response. Various methods for measuring affinity for an antigen that are well known in the pertinent art can be used, and examples thereof include surface plasmon resonance (SPR) technology.

The present invention further provides a polynucleotide encoding the heavy chain variable region and the light chain variable region of the monoclonal antibody or an antigen-binding fragment thereof.

The polynucleotide encoding the light chain and heavy chain of the monoclonal antibody of the present invention or an antigen-binding fragment thereof may be varied within the coding region without altering the amino acid sequence of the antibody's light chain and heavy chain encoded by the coding region, due to the degeneracy of codons or by considering the preferred codons in the host organism in which the light and heavy chains of the human antibody or fragments thereof are to be expressed. Moreover, various modifications or alterations can be made in parts other than the coding region, as long as they do not affect the expression of the gene, and it is easily understood by those skilled in the pertinent art that such modified genes also fall within the scope of the present invention. In other words, as long as the polynucleotide of the present invention encodes a protein with equivalent activity, one or more nucleotide bases in the sequence can be substituted, deleted, inserted, or altered by any combination of these modifications, and these variations are also included within the scope of the present invention. The sequence of such polynucleotides may be single-stranded or double-stranded, and may be in the form of a DNA molecule or an RNA (mRNA) molecule.

The present invention further provides a recombinant expression vector comprising a polynucleotide encoding the heavy chain and the light chain variable regions of the monoclonal antibody of the present invention or an antigen-binding fragment thereof.

In the present invention, the term "expression vector" refers to a genetic construct used to express a target gene in a host cell. This includes plasmid vectors and viral vectors such as cosmid vectors, adenovirus vectors, retrovirus vectors, and adeno-associated virus (AAV) vectors, and includes essential regulatory elements that are operably linked to ensure the expression of a gene insert. In the present invention, "operably linked" means that the nucleic acid regulatory sequence for expression and the polynucleotide encoding the desired protein are functionally connected so that the desired function can be performed. The operable link with the expression vector can be achieved using recombinant DNA techniques that are well known in the pertinent art, and site-specific DNA cleavage and ligation can be easily carried out using enzymes that are commonly known in the field of pertinent art.

The suitable expression vector of the present invention may include not only expression regulatory elements such as a promoter, initiation codon, termination codon, polyadenylation signal, and enhancer, but also a signal sequence for membrane targeting or secretion. The initiation codon and termination codon are generally considered part of the nucleotide sequence encoding the immunogenic target protein and must function appropriately when the genetic construct is introduced into an organism. Furthermore, these codons should be in-frame with the coding sequence. The promoter can be constitutive or inducible. Prokaryotic cells may use promoters such as Lac, Tac, T3, or T7, but it is not limited to them. Eukaryotic cells may use promoters such as the simian virus 40 (SV40) promoter, mouse mammary tumor virus (MMTV) promoter, human immunodeficiency virus (HIV) promoter, the long terminal repeat (LTR) promoter of HIV, Moloney virus promoter, cytomegalovirus (CMV) promoter, Epstein-Barr virus (EBV) promoter, as well as promoters derived from β-actin, human hemoglobin, human muscle creatine, and human metallothionein, though it is not limited to them.

The expression vector may also include a selection marker to select the host cell containing the vector. The selection marker is used to screen for cells transformed with the vector, and markers that confer selectable phenotypes such as drug resistance, nutritional requirements, resistance to cytotoxic agents, or surface protein expression may be used. Only the cells expressing the selection marker survive in an environment treated with a selective agent, thus allowing for the selection of the transformed cells.

Additionally, if the vector is a replicable expression vector, it may include a replication origin, which is a specific polynucleotide in which replication is initiated. Vectors that can be inserted into the genome of host cells, such as viral (for example, baculovirus) or phage vectors, and retrovirus vectors, can also be used. Vectors that express full antibodies or antibody fragments may either be in a vector system in which the light chain and heavy chain are expressed simultaneously in a single vector, or in a system in which the light chain and heavy chain are expressed in separate vectors. In the latter case, the two vectors are introduced into host cells through co-transformation and targeted transformation, and cells transformed with the vector containing the light chain (or heavy chain) are selected. The selected cells expressing the light chain are then transformed again with the vector containing the heavy chain (or light chain) to ultimately select cells that express both the light chain and heavy chain.

To produce antibodies in the Fab form, a vector is used that inserts genes encoding the amino acids of the variable region (VL) and constant region (CL) of the human light chain, as well as the variable region (VH) and the first constant region domain (CH1) of the human heavy chain.

The present invention further provides a non-human transformant that has been transformed with the expression vector of the present invention.

Suitable host cells for the vector may include prokaryotic cells such as *Escherichia coli, Bacillus subtilis, Streptomyces* sp., *Pseudomonas* sp., *Proteus mirabilis,* or *Staphylococcus* sp.; fungi such as *Aspergillus* sp.; yeasts such as *Pichia pastoris, Saccharomyces cerevisiae, Schizosaccharomyces* sp., and *Neurospora crassa;* other lower eukaryotic cells; and cells from higher eukaryotes such as insect cells or plant cells. Additionally, mammalian cells derived from COS-7, BHK, CHO, CHO-K1, DXB-11, DG-44, CHO/-DHFR, CV1, HEK293, BHK, TM4, VERO, HELA, MDCK, BRL3A, W138, Hep G2, SK-Hep, MMT, TRI, MRC 5, FS4, 3T3, RIN, A549, PC12, K562, PER.C6, SP2/0, NS-0, U20S, or HT1080 cells may also be used, but are not limited to them.

In the present invention, "transformation of a host cell" refers to any method of introducing nucleic acids into an organism, cell, tissue, or organ, and it can be performed using standard techniques suitable for the host cell as known in the pertinent art. These methods include electroporation, protoplast fusion, calcium phosphate (CaPO₄) precipitation, calcium chloride (CaCl₂) precipitation, agitation using silicon carbide fibers, *Agrobacterium-mediated* transformation, PEG (Polyethylene glycol), dextran sulfate, lipofectamine, and drying/inhibition-mediated transformation methods, but are not limited to them.

The present invention further provides a method for producing a monoclonal antibody specifically binding to claudin-18.2 or an antigen-binding fragment thereof, including:
(a) culturing the transformant to prepare a culture solution; and
(b) purifying the monoclonal antibody of the present invention or an antigen-binding fragment thereof from the culture solution obtained in step (a).

In the production method described above, the culturing of the transformant can be carried out using an appropriate medium and culturing conditions that are known to those skilled in the pertinent art. This culturing process can be easily adjusted according to the strain or cell type selected by those skilled in the pertinent art.

Furthermore, the antibody obtained by culturing the transformant may be used in an unpurified state, and impurities can be removed by various conventional methods such as centrifugation or ultrafiltration. The resulting product can then be purified by dialysis, salt precipitation, chromatography, or combinations of these methods. Among them, affinity chromatography is the most commonly used, but ion-exchange chromatography, size-exclusion chromatography, hydrophobic interaction chromatography, and hydroxylapatite chromatography can also be used. Antibodies prepared by those methods are antibodies with increased affinity for the antigen.

The present invention further provides a pharmaceutical composition for treating cancer, which comprises the monoclonal antibody specifically binding to claudin-18.2 or an antigen-binding fragment thereof as an active ingredient.

The monoclonal antibody or an antigen-binding fragment thereof is as described above.

The pharmaceutical composition of the present invention, when administered to a subject, inhibits the growth of tumors in the subject.

The term "treatment of cancer" in the present invention refers to any action in which the symptoms of cancer are improved or beneficially altered by the administration of the composition.

In the pharmaceutical composition according to the present invention, the cancer may be any cancer selected from the group consisting of pancreatic cancer, esophageal cancer, ovarian cancer, lung cancer, gastric cancer, colorectal cancer (e.g., colon cancer, rectal cancer), liver cancer, biliary tract cancer, gallbladder cancer, breast cancer, kidney cancer, mesothelioma, head and neck cancer, bladder cancer, cervical cancer, endometrial cancer, fallopian tube cancer, gastrointestinal cancer, hematologic cancers (e.g., leukemia, lymphoma, or myeloma), oropharyngeal cancer, melanoma, primary peritoneal cancer, salivary gland cancer, sarcoma, thyroid cancer, glioblastoma, and prostate cancer, but is not limited to them.

The pharmaceutical composition of the present invention may further comprise a pharmaceutically acceptable carrier, and the carrier may include a non-naturally occurring carrier.

In the present invention, the term "pharmaceutically acceptable carrier" refers to a carrier or diluent that does not stimulate a living organism and does not interfere with the biological activity and properties of the administered compound. Pharmaceutically acceptable carriers for compositions formulated in liquid solutions include those that are sterile and biologically compatible, such as saline, sterile water, Ringer's solution, buffered saline, albumin injection, dextrose solution, maltodextrin solution, glycerol, ethanol, or mixtures of one or more of these components. Additionally, conventional additives such as antioxidants, buffers, bacteriostats, etc., may be added as needed. Furthermore, diluents, dispersing agents, surfactants, binders, and lubricants may be added to formulate injectable preparations such as aqueous solutions, suspensions, emulsions, as well as pills, capsules, granules, or tablets.

The composition for cancer treatment comprising the monoclonal antibody of the present invention and a pharmaceutically acceptable carrier can be applied in any formulation containing it as the active ingredient, and can be manufactured into oral or parenteral formulations. The pharmaceutical formulations of the present invention include those suitable for administration by oral, rectal, nasal, topical (including buccal and sublingual), subcutaneous, vaginal, or parenteral (including intramuscular, subcutaneous, and intravenous) routes, or in a form suitable for inhalation administration.

Examples of the oral formulations containing the pharmaceutical composition of the present invention as the active ingredient include tablets, troches, lozenges, aqueous or oily suspensions, powders or granules for preparation, emulsions, hard or soft capsules, syrups, or elixirs. To formulate the compositions into tablets or capsules, excipients such as binders like lactose, sucrose, sorbitol, mannitol, starch, amilopectin, cellulose, or gelatin, diluents like dicalcium phosphate, disintegrants like corn starch or sweet potato starch, and lubricants such as magnesium stearate, calcium stearate, sodium stearyl fumarate, or polyethylene glycol waxes can be included. In the case of capsule formulations, in addition to the above-mentioned substances, liquid carriers such as fatty oils may also be included.

Examples of the parenteral formulations comprising the pharmaceutical composition of the present invention as the active ingredient include injectable forms such as subcutaneous, intravenous, or intramuscular injections, suppositories for rectal administration, or aerosol formulations for respiratory inhalation used as a spray. To formulate injectable formulations, the composition of the present invention can be mixed with stabilizers or buffers in water to prepare a solution or suspension, which can then be formulated for unit doses in ampoules or vials. To formulate for rectal administration, suppositories or enema compositions containing conventional suppository bases such as cocoa butter or other glycerides can be prepared. In the case of spray formulations like aerosol, propellants may be combined with the additives so that the concentrate dispersed in water or wet powder can be dispersed.

Additionally, the pharmaceutical composition of the present invention can be administered in pharmaceutically effective amount.

In the present invention, the term "pharmaceutically effective amount" refers to an amount that is sufficient to treat the disease with a reasonable benefit/risk ratio applicable to medical treatment. The effective dose level can be determined based on factors such as the type of subject, severity, age, gender, type of cancer, drug activity, sensitivity to the drug, time of administration, route of administration, excretion rate, duration of treatment, co-administered drugs, and other factors well known in the medical field. The composition of the present invention can be administered either as a single agent or in combination with other therapeutic agents, and it can be administered sequentially or simultaneously with conventional therapies. It may also be administered in a single dose or multiple doses. Considering all of these factors, it is important to administer the minimum amount that achieves maximum effect without side effects, and this can be easily determined by one skilled in the pertinent art.

The pharmaceutical composition of the present invention may additionally contain one or more known active ingredients with anti-cancer effects. These known anti-cancer active ingredients are preferably chemotherapeutic agents or immune checkpoint inhibitors, but are not limited to them.

The chemotherapeutic agents can be one or more selected from the group consisting of alkylating anti-cancer agents such as carboplatin and paclitaxel, metabolic antagonist-based anti-cancer agents such as gemcitabine, anthracycline-based anti-cancer agents such as doxorubicin, and proteasome inhibitor-based anti-cancer agents such as bortezomib, but are not limited to them. The immune checkpoint inhibitors can target one or more selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, Transferrin receptor, c-Met, EGFR, HER2, KDR, PDGFRa, NRP1, and MARCO, but are not limited to them.

The present invention further provides a method for treating cancer, comprising administering the pharmaceutical composition for treating cancer to a subject in need thereof.

In the present invention, the term "administration" means introducing the pharmaceutical composition of the present invention to a patient by any appropriate method. The route of administration of the composition of the present inventio can be any route that can reach the target tissue, and specifically, it can be administered via various routes including oral and parenteral routes such as oral, rectal, topical, intravenous, intraperitoneal, intramuscular, intra-arterial, transdermal, intranasal, inhalation, intraocular, or intradermal administration, in a conventional manner.

The treatment method of the present invention includes administering the pharmaceutical composition for cancer treatment of the present invention in an effective amount. A suitable total daily dose can be determined by the attending physician within the proper medical judgment range, which is obvious to those skilled in the pertinent art. The specific therapeutic effective dose for a particular patient may vary depending on factors such as the type and degree of response to be achieved, whether another formulation is used, the specific composition, the patient's age, weight, general health condition, sex, diet, administration time, route of administration, excretion rate of the composition, treatment duration, and other factors well-known in the pharmaceutical field, including drugs used or co-administered with the specific composition. Therefore, the effective amount of the composition for cancer prevention or treatment suitable for the purposes of the present invention should be determined considering the above factors.

Furthermore, the subject refers to any animal that may have tumor development and develop diseases such as angiogenesis due to excessive activation of claudin-18.2, and it includes humans, primates, as well as livestock such as cattle, pigs, sheep, horses, dogs, and cats. Preferably, the subject is a non-human mammal, but is not limited thereto.

The present invention further provides a cancer diagnostic composition comprising the monoclonal antibody specifically binding to claudin-18.2 or an antigen-binding fragment thereof, and a cancer diagnostic kit comprising the cancer diagnostic composition.

The monoclonal antibody or an antigen-binding fragment thereof and the cancer-related information are described above. By using the diagnostic composition comprising the claudin-18.2-specific monoclonal antibody of the present invention or an antigen-binding fragment thereof, it is possible to diagnose diseases related to the expression and degree of expression of claudin-18.2, such as cancer.

The method for diagnosing cancer includes reacting the claudin-18.2-specific monoclonal antibody of the present invention with a biological sample isolated from a subject suspected of having cancer, and detecting antigen-antibody complex formation, which can provide diagnostic information for cancer.

In the present invention, the term "biological sample" refers to a sample that can include tissue, cells, whole blood, serum, plasma, tissue biopsy samples (e.g., brain, skin, lymph nodes, spinal cord, etc.), cell culture supernatant, lysed eukaryotic cells, and bacterial expression systems, but is not limited to them. These biological samples can be reacted, either in modified form or unmodified form, with the antibody of the present invention to determine the presence of claudin-18.2 or the existence of cancer.

In the present invention, the term "antigen-antibody complex" refers to a binding product between the claudin-18.2 protein antigen in a sample and the monoclonal antibody of the present invention that recognizes the claudin-18.2 protein antigen or an antigen-binding fragment thereof. The formation of such antigen-antibody complex can be measured using conventional immunoassay methods, including but not limited to radioimmunoassay, radioimmunoprecipitation, immunoprecipitation, immunohistochemistry, ELISA (enzyme-linked immunosorbent assay), capture-ELISA, inhibition or competition assays, sandwich assays, flow cytometry, and fluorescence immunoassay.

By analyzing the intensity of the final signal from the immunoassay process, cancer can be diagnosed. Specifically, if the claudin-18.2 protein of the present invention is overexpressed in a biological sample isolated from a suspected subject, and the signal is stronger than that from a biological sample isolated from a normal subject, the subject can be diagnosed with cancer.

Moreover, the kit according to the present invention includes the antibody against claudin-18.2 of the present invention, and cancer can be diagnosed by analyzing the signal generated when the sample and the antibody react. In this case, the signal can be produced by an enzyme conjugated to the antibody (e.g., alkaline phosphatase, β-galactosidase, horseradish peroxidase, luciferase, or cytochrome P450, etc.). As for the enzyme substrate, when alkaline phosphatase is used as the enzyme, color reaction substrates such as bromochloroindolyl phosphate (BCIP), nitro blue tetrazolium (NBT), naphthol-AS-B1-phosphate, and ECF (enhanced chemifluorescence) can be used. When horseradish peroxidase is used as the enzyme, substrates such as chloronaphthol, aminoethylcarbazole, diaminobenzidine, D-luciferin, lucigenin (bis-N-methylacridinium nitrate), resorufin benzyl ether, luminol, Amplex Red reagent (10-acetyl-3,7-dihydroxyphenoxazine), HYR (p-phenylenediamine-HCl and pyrocatechol), TMB (tetramethylbenzidine), ABTS (2,2'-Azine-di[3-ethylbenzthiazoline sulfonate]), o-phenylenediamine (OPD), and naphthol/pyronine, glucose oxidase, t-NBT, or m-PMS (phenazine methosulfate) can be used, though they are not limited to the listed examples.

Additionally, the kit according to the present invention may include a label that generates a detectable signal, and the label can be a chemical substance (e.g., biotin), an enzyme (e.g., alkaline phosphatase, β-galactosidase, horseradish peroxidase, and cytochrome P450), a radioactive substance (e.g., ¹⁴C, ¹²⁵I, ³²P and ³⁵S), a fluorescent substance (e.g., fluorescein), a luminescent substance, a chemiluminescent substance, or FRET (fluorescence resonance energy transfer), though it is not limited to them.

The measurement of enzyme activity or the measurement of the signal used for cancer diagnosis can be conducted using various methods that are known in the pertinent art. Through this, the expression of claudin-18.2 can be analyzed either qualitatively or quantitatively.

The present invention further provides an antibody-drug conjugate, in which a drug is bound to the monoclonal antibody specifically binding to claudin-18.2 or an antigen-binding fragment thereof.

In the present invention, the term "drug" refers to a compound that can bind to the monoclonal antibody specific to claudin-18.2 or an antigen-binding fragment thereof but can be separated from the antibody or an antigen-binding fragment thereof under acidic conditions, and exhibits therapeutic effects on the target cells.

The drugs that can be used in the antibody-drug conjugate according to the present invention include any compound, portion, or derivative that has cytotoxic or antiproliferative effects, such as (i) chemotherapy agents that can function as microtubule inhibitors, mitosis inhibitors, topoisomerase inhibitors, or DNA intercalators; (ii) enzymatically active protein toxins; and (iii) radioactive isotopes (radiation radionuclides), and one or more of these compounds can be used.

Non-limiting examples of such drugs include maytansinoid, auristatin, dolastatin, trichothecene, CC1065 (Rachelmycin), calicheamicin and other enediyne antibiotics, taxanes, anthracyclines, methotrexate, Adriamycin, vindesine, vinca alkaloids (vincristine, vinblastine, etoposide), doxorubicin, melphalan, mitomycin C, chlorambucil, daunorubicin, daunomycin, etoposide, teniposide, carminomycin, aminopterin, dactinomycin, mitomycin-related compounds, bleomycin-related compounds, esperamicin-related compounds, 5-fluorouracil, melphalan, other nitrogen mustards and their stereoisomers, homologs, isomers, or derivatives, cisplatin and its homologs, and other intercalating enzymes and fragments, for example, nucleases, antibiotics, toxins (enzymatically active bacterial, fungal, plant, or animal-origin toxins or small molecule toxins), cisplatin, CPT-11, doxorubicin, paclitaxel, docetaxel, and other various anti-tumor or anti-cancer agents, though the drugs are not limited to them. Additionally, examples thereof include the radioactive isotopes (radiation radionuclides) include ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, and ¹⁸⁶Re, though not limited to them. Furthermore, the drugs can be selected from the group consisting of immune modulatory compounds, anti-viral agents, anti-bacterial agents, anti-fungal agents, anti-parasitic agents, microRNAs (miRNA), siRNAs, shRNAs to suppress the expression of specific oncogenes, and a combination thereof.

The present invention further provides a chimeric antigen receptor (CAR) protein comprising: i) the monoclonal antibody that specifically binds to claudin-18.2; ii) a transmembrane domain; and iii) an intracellular signaling domain characterized in that it induces T cell activation upon binding of the antibody of i) to the antigen.

In the present invention, the CAR (chimeric antigen receptor) protein can be defined by being consisted of the monoclonal antibody of the present invention, a known transmembrane domain, and an intracellular signaling domain.

In the present invention, the term "CAR (chimeric antigen receptor)" refers to a receptor that does not naturally exist but can provide immune effector cells with specificity to a particular antigen. Typically, a CAR refers to a receptor used to transplant the specificity of a monoclonal antibody into T cells. A CAR generally consists of an extracellular domain, a transmembrane domain, and an intracellular domain. The extracellular domain includes the antigen recognition region, which, in the present invention, is the antibody specific to claudin-18.2. The antibody specific to claudin-18.2 is as described earlier, and the antibody used in the CAR is preferably in the form of an antibody fragment, and more preferably in the form of Fab or scFv, though not limited to them.

Additionally, the transmembrane domain of the CAR is connected to the extracellular domain and may be derived from naturally occurring or synthetic sources. If derived from naturally occurring sources, it may come from membrane-binding or transmembrane proteins, such as the alpha, beta, or zeta chains of the T cell receptor, or various other transmembrane regions from proteins like CD28, CD3 epsilon, CD45, CD4, CD5, CD8, CD9, CD16, CD22, CD33, CD37, CD64, CD80, CD86, CD134, CD137, and CD154. The sequence of these transmembrane domains can be obtained from known literature or sources of transmembrane proteins, but it is not limited to them. Additionally, if the transmembrane domain is synthetic, it may primarily contain hydrophobic amino acid residues, such as leucine and valine, and an example could be a transmembrane domain having synthesized triplet of phenylalanine, tryptophan, and valine, though not limited thereto.

In the CAR of the present invention, the intracellular domain is part of the CAR present inside the cell and is connected to the transmembrane domain. The intracellular domain of the present invention may include an intracellular signaling domain, which is characterized by activating T cells, preferably inducing T cell proliferation, upon antigen binding to the antigen-binding site of the CAR. The intracellular signaling domain is not particularly limited in type, as long as it transmits a signal that leads to T cell activation upon binding of the antibody to the extracellular antigen-binding site. Various types of intracellular signaling domains may be used, for example, an immunoreceptor tyrosine-based activation motif (ITAM), which may be derived from CD3 zeta (ξ, zeta), FcR gamma, FcR beta, CD3 gamma, CD3 delta, CD3 epsilon, CD5, CD22, CD79a, CD79b, CD66d, or FcεRIγ, though not limited to them.

Furthermore, the intracellular domain of the CAR in the present invention may preferably include, but is not limited to, a costimulatory domain, in addition to the intracellular signaling domain. The costimulatory domain refers to a portion of the CAR's intracellular region including intracellular domains of costimulatory molecules, which transmits signals to the T cells in addition to the signals from the intracellular signaling domain. The costimulatory molecules are cell surface molecules necessary for sufficient lymphocyte responses to antigens. Examples thereof include CD27, CD28, 4-1BB, OX40, CD30, CD40, PD-1, ICOS, LFA-1 (lymphocyte function-associated antigen-1), CD2, CD7, LIGHT, NKG2C, or B7-H3, though not limited to them. The costimulatory domain may be an intracellular part of the molecule selected from the group consisting of those costimulatory molecules and a combination thereof.

Furthermore, optionally, a short oligopeptide or polypeptide linker can connect the intracellular domain and the transmembrane domain of the CAR. The linker may be included in the CAR of the present invention, and as long as it can induce T cell activation through the intracellular domain upon binding of an antigen to the antibody located extracellularly, its length is not particularly limited.

The present invention still further provides a multi-specific antibody comprising the monoclonal antibody that specifically binds to claudin-18.2 or an antigen-binding fragment thereof.

In the present invention, the multi-specific antibody may preferably be a bispecific antibody, but it is not limited thereto.

The multi-specific antibody according to the present invention is preferably in the form in which the anti-claudin-18.2 antibody according to the present invention is bound to an antibody that has binding property to an immune effector cell-specific target molecule or a fragment thereof. The immune effector cell-specific target molecule is preferably selected from PD-1, PD-L1, CTLA-4, TIM-3, TIGIT, BTLA, KIR, A2aR, VISTA, B7-H3, TCR/CD3, CD16 (FcγRIIIa), CD44, CD56, CD69, CD64 (FcγRI), CD89, and CD11b/CD18 (CR3), although not limited to them.

Multi-specific antibody is an antibody that recognizes multiple (two or more) epitopes simultaneously on the same or two or more different antigens. Antibodies within the multi-specific antibody category can be classified as scFv-based antibodies, Fab-based antibodies, and IgG-based antibodies. Multi-specific antibodies, for example bispecific antibodies, can be more effective than suppressing or amplifying a single signal because they can suppress or amplify two signals simultaneously. Compared to treating each signal separately with its own signal inhibitors, bispecific antibodies may allow for lower doses and simultaneous suppression/amplification of both signals in the same time and space.

The methods for producing bispecific antibodies are well known. Traditionally, recombinant production of bispecific antibodies is based on the co-expression of two immunoglobulin heavy and light chain pairs with different specificities under conditions in which the two heavy chains have different specificities.

In the case of a bispecific antibody based on scFv, different scFv light chain variable regions (VL) and heavy chain variable regions (VH) can be combined to create hybrid scFvs in a heterodimeric form, which can be used to make diabodies (Holliger et al., Proc. Natl. Acad. Sci. U.S.A., (1993) 90: 6444). Alternatively, different scFvs can also be linked to create tandem scFvs, and Fab's CH1 and CL can be expressed at the ends of each scFv to produce heterodimeric mini-antibodies (Muller et al., FEBS Lett., (1998) 432: 45). Additionally, by substituting certain amino acids in the homodimeric CH3 domain of Fc to create a heterodimeric structure in the 'knob into hole' form, the modified CH3 domains can be expressed at the ends of different scFvs, thus producing mini antibodies in the heterodimeric scFv form (Merchant et al., Nat. Biotechnol., (1998) 16: 677).

In the case of a bispecific antibody based on Fab, individual Fab's against specific antigens can be combined into a heterodimeric Fab form using disulfide bonds or linking agents. Alternatively, scFvs against different antigens can be expressed at the termini of the heavy or light chain of a specific Fab, which can result in an antigen valency of two. Additionally, by introducing a hinge region between the Fab and scFv, a homodimeric form with an antigen valency of 4 can be produced.

Additionally, a method of producing a bispecific antibody (bibody) with an antibody valency of 3 by fusing scFvs against different antigens to the termini of the heavy and light chain of Fab, a trispecific antibody (bibody) by fusing different scFvs to the termini of the Fab's light and heavy chains to have an antigen valency of 3, and a trispecific antibody known as F(ab')₃ in a simple form by chemical conjugation of three different Fabs is well-known in the pertinent art.

In the case of bispecific antibodies based on IgG, Trion Pharma has reported a method for producing bispecific antibodies by re-crossbreeding mouse and rat hybridomas to create hybrid hybridomas, also known as quadromas. Additionally, a method for producing bispecific antibodies has been reported in which the light chain portion is shared, and different heavy chains are used by modifying certain amino acids in the Fc CH3 homodimeric domain to create a heterodimeric form, referred to as the "Holes and Knob" method (Merchant et al., 1998). Furthermore, a method has been reported in which two different scFvs are fused and expressed at the constant domains of IgG's light and heavy chain, instead of the variable domains, to create a homodimeric form of (scFv)₄-IgG. Furthermore, ImClone has reported creating a bispecific antibody by fusing only the single variable domain against mouse platelet-derived growth factor receptor-alpha (PDGFR-α) to the amino terminus of the light chain of the chimeric monoclonal antibody IMC-1C11, which targets human VEGFR-2. Rossi et al. have used a "dock and lock" method involving the dimerization and docking domain of protein kinase A (PKA) R subunit and the PKA anchoring domain to produce an antibody with multiple antigen-binding sites targeting CD20 (Rossi et al., Proc. Natl. Acad. Sci. U.S.A., (2006) 103: 6841).

Hereinbelow, the present invention is explained in detail through the Examples. However, the following examples are provided only for exemplification of the present invention, and the present invention is not limited to the contents of those examples.

### EXAMPLES

### Example 1. Selection of antibodies specific to claudin-18.2 (CLDN18.2)

The gene for CLDN18.2 (R&D, #RDC2149) was overexpressed in the HEK293E animal cell line to establish a cell line. The CLDN18.2-VLPs antigen (Cusabio, #CSB-MP005498HU(A5)) was coated onto Immunosorb tubes and blocked. After infecting *Escherichia coli* with human scFv library phage (Y-Biologics Co., Ltd.), the resulting bacteria were cultured at 30°C for 16 hours. The culture broth was centrifuged and the supernatant was concentrated using polyethylene glycol (PEG), and then dissolved in PBS (phosphate buffered saline) to prepare the human antibody library phage.

The prepared library phage was added to the immunosorb tube and incubated at room temperature for 2 hours. After washing with 1x PBST and 1x PBS, only the scFv-phages specifically bound to the antigen were eluted by treating sequentially with 100 mM TAE and Tris-HCl (pH 7.5) solutions. Panning and ELISA were then performed, and three positive phage clones specifically binding to CLDN18.2 were selected. The scFv clones were subsequently converted into IgG form for use in experiments.

The sequences of the heavy and light chain CDRs of the selected monoclonal antibodies, as well as the sequences of the heavy and light chain variable regions containing them, are provided in Tables 1 and 2 below.

**[Table 1]**

| Sequences of heavy chain and light chain CDRs of monoclones 0058-001, 0058-002, and 0058-003 | | | | | | |
|---|---|---|---|---|---|---|
| Clone name | Heavy chain | | | Light chain | | |
| | CDR1 | CDR2 | CDR3 | CDR1 | CDR2 | CDR3 |
| 0058-001 | GFTFSSYA (SEQ ID NO: 1) | ISGSGGS T (SEQ ID NO: 2) | AKEGVWWLR YFDH (SEQ ID NO: 3) | SNDVGGYT Y (SEQ ID NO: 5) | DVS | SSYAGSYT WV (SEQ ID NO: 6) |
| 0058-002 | GFTFSSYA (SEQ ID NO: 1) | ISGSGGS T (SEQ ID NO: 2) | ARGLGYYYY GMDV (SEQ ID NO: 8) | QTVSSW (SEQ ID NO: 10) | AAS | QQYHSFPPT (SEQ ID NO: 11) |
| 0058-003 | GFTFSSYA (SEQ ID NO: 1) | , ISGSGGS T (SEQ ID NO: 2) | , ARGAASSYYF DY (SEQ ID NO: 13) | AGVRNY (SEQ ID NO: 15) | GAF | QQYHSFPPT (SEQ ID NO: 11) |

**[Table 2]**

| Sequences of variable regions of heavy chains and light chains of monoclones 0058-001, 0058-002, and 0058-003 | | |
|---|---|---|
| Clone name | Heavy chain variable region | Light chain variable region |
| 0058-001 | QVQLVESGGGLVQPGGSLRLSCA ASGFTFSSYAMSWVRQAPGKGLE WVAAISGSGGSTYFADSVRGRFTI SRDNPQSTLYLQMNSLRAEDTAV YYCAKEGVWWLRYFDHWGQGTP ITVSS (SEQ ID NO: 4) | QSALTQPRSVSGSPGQSVTISCTGT SNDVGGYTYVSWYQQHPGKAPK LMIYDVSKRPSGVPDRFSGSKSGN TASLTISGLQAEDEADYYCSSYAG SYTWVFGGGTKLTVL (SEQ ID NO: 7) |
| 0058-002 | QMQLVESGGGLVQPGGSLRLSCA ASGFTFSSYAMSWVRQAPGKGLE WVSAISGSGGSTYYADSVKGRFTI SRDNSKNTLYLQMNSLRAEDTAV YYCARGLGYYYYGMDVWGQGTT VTVSS (SEQ ID NO: 9) | DIQMTQSPSSLSASVGDRVTITCRA SQTVSSWLAWYQQKPGKAPKLLI YAASSLQSGVPSRFSGSGSGTEFTL TISSLQPEDFGTYYCQQYHSFPPTF GGGTKVGIK (SEQ ID NO: 12) |
| 0058-003 | VTVSS (SEQ ID NO: 9) QVQLVQSGGGLVQPGGSLRLSCA ASGFTFSSYAMSWVRQAPGKGLE WVSAISGSGGSTYYADSVKGRFTI SRDNSKNTLYLQMNSLRAEDTAV YYCARGAASSYYFDYWGLGTTVT VSS (SEO ID NO: 14) | DIQMTQSPSSLAAYVGDTVTITCR ASAGVRNYLAWVQQKPGKAPRSL IYGAFKLQSGVPSRFSGSGSGTEFT LTISSLQPEDFGTYYCQQYHSFPPT FGGGTKVDIK (SEQ ID NO: 16) |
| Underlined: CDR | | |

### Example 2. Characteristics of selected CLDN18.2-specific monoclonal antibodies

### 2-1. Non-specificity of CLDN18.2 monoclonal antibodies based on flow cytometry analysis

A non-specific binding assay was performed using the HEK293E cell line. The expression of human CLDN18.2 was determined using anti-CLDN18.2 (CUSABIO, #CSB-RA005498A1HU).

HEK293E cells that do not express human CLDN18.2 were prepared to a concentration of 0.5 × 10⁶ cells per sample. The selected antibodies 0058-001, 0058-002, and 0058-003 were diluted to a concentration of 7.5 µg/mL and incubated with the prepared cells at 4°C for 30 minutes. Subsequently, the cells were washed three times with PBS (Welgene, #LB001-02) containing 2% fetal bovine serum (FBS) and incubated with FITC (fluorescein isothiocyanate)-labeled anti-human IgG antibody (Vectorlabs, #FI-3000) at 4°C for 30 minutes. The cells were washed again as described above. The cells were resuspended in 0.2 mL of PBS containing 2% FBS (Thermo, #26140-079), and binding property was analyzed using a CytoFlex flow cytometer (Beckman Coulter, USA).

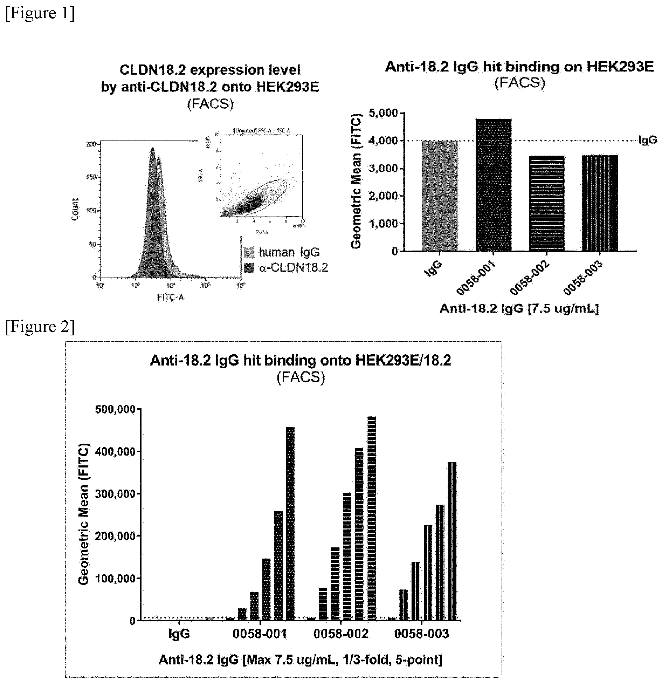

The results of the analysis indicated that none of the selected antibodies 0058-001, 0058-002, or 0058-003 bound to the HEK293E cell line that did not express CLDN18.2 (Figure 1).

### 2-2. Specificity of CLDN18.2 monoclonal antibodies based on flow cytometry analysis

Human CLDN18.2-overexpressing HEK293E/18.2 cells and CHO-K1/18.2 cells were separately prepared to a concentration of 0.5 × 10⁶ cells per sample. The anti-CLDN18.2 antibodies were serially diluted and incubated with the prepared cells at 4°C for 30 minutes. After incubation, the cells were washed three times with PBS containing 2% fetal bovine serum (FBS), followed by incubation with FITC-labeled anti-human IgG antibody at 4°C for 20 minutes. The cells were then washed as described above. The cells were resuspended in 0.5 mL of PBS containing 2% FBS, and binding property was analyzed using a CytoFlex flow cytometer.

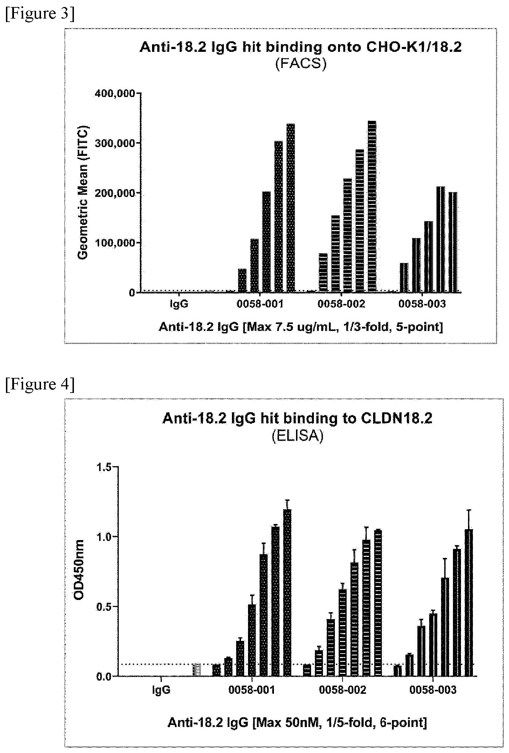

The analysis results indicated that the selected anti-CLDN18.2 antibodies 0058-001, 0058-002, and 0058-003 bound to the HEK293E/18.2 and CHO-K1/18.2 cell lines in a concentration-dependent manner, in which CLDN18.2 was highly expressed (Figures 2 and 3).

### Example 3. Analysis of binding properties of selected CLDN18.2-specific monoclonal antibodies against CLDN18 isoforms

Claudin-18.1 is an antigen expressed in normal lung and gastric epithelium, and CLDN18.1/18.2 isoforms are produced depending on which ATG in the first exon of the claudin-18 gene is used as the start codon, and since 92% of the protein sequence is identical, the selective binding property to isoforms was analyzed to select an antibody that specifically binds to claudin-18.2 and not to claudin-18.1.

3-1. Binding specificity analysis of antibodies to CLDN18 isoforms by ELISA CLDN18.1 (EUPROTEIN, #EPY255141) and CLDN18.2 (CUSABIO, #CSB-MP005498HU(A5)) proteins were each prepared at a concentration of 1 µg/mL using a dilution buffer (4% skim milk/0.05% tween-20/PBS), and 100 µL of each was added to individual wells of an immunoassay plate (Thermo, #439454) and incubated statically at 4°C for more than 16 hours. After three washes with 300 µL of wash buffer (0.05% tween-20/PBS), 200 µL of blocking buffer (4% skim milk/0.05% tween-20/PBS) was added to the wells and incubated for 1 hour at room temperature. After three washes, 100 µL of the anti-CLDN18.2 antibodies, each prepared by serial dilution, was added to each well and incubated at room temperature for 1 hour with rocking. After three washes, 100 µL of anti-human kappa-HRP (Sigma, #A7164) antibody, prepared at dilution ratio of 1:3,000, was added to each well and incubated at room temperature for 1 hour. After three washes, 100 µL of TMB substrate (Sigma, #T0440) was added to each well and incubated in the dark at room temperature, and when color development was observed, 50 µL of stop solution (1N H₂SO₄) was added to stop the reaction. Absorbance was measured using a microplate reader (Promega, GM3000).

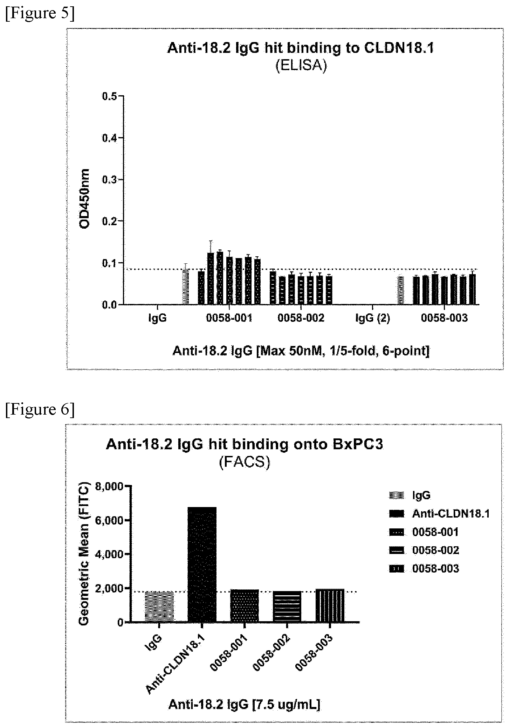

The analysis results indicate that the selected antibodies 0058-001, 0058-002, and 0058-003 bound to the CLDN18.2 antigen (Figure 4) but did not bind at all to the CLDN18.1 antigen (Figure 5), showing their selective binding to the CLDN18.2 antigen.

### 3-2. Binding specificity analysis of antibodies to CLDN18 isoforms using flow cytometry

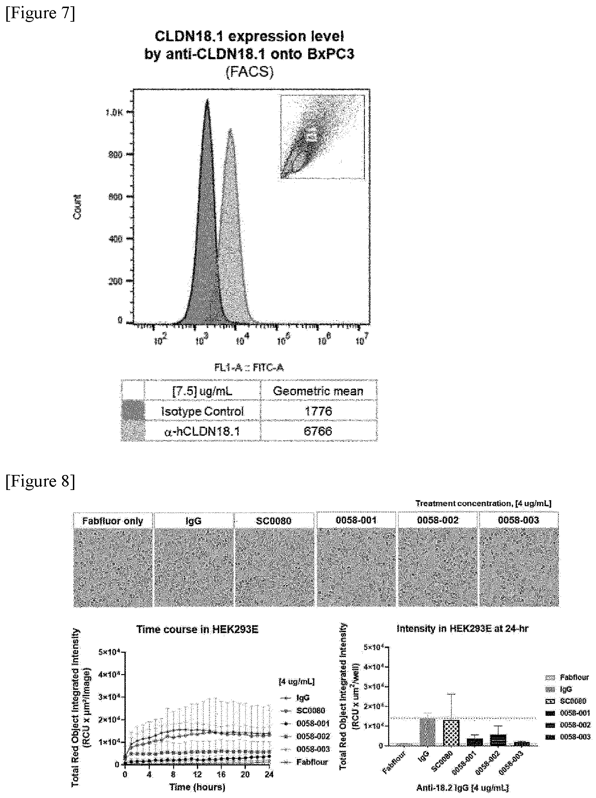

The BxPC3 cells endogenously expressing human CLDN18.1 (Figure 7) were prepared to a concentration of 0.5 × 10⁶ cells per sample, and incubated with 50 nM of anti-CLDN18.1 antibody (Cusabio, #CSB-RA005498A2HU) or the anti-CLDN18.2 antibody of the present invention at 4°C for 30 minutes. Afterward, the cells were washed three times with PBS containing 2% fetal bovine serum (FBS), and reacted with FITC-labeled anti-human IgG antibody at 4°C for 30 minutes, followed by the same washing procedure as above. The cells were resuspended in 0.2 mL of PBS containing 2% FBS, and binding property was analyzed using a CytoFlex flow cytometer.

The results showed that the anti-CLDN18.1 antibody bound to the BxPC3 cell line, while all the selected anti-CLDN18.2 antibodies did not bind to the BxPC3 cell line (Figure 6). These results indicate that the selected antibodies 0058-001, 0058-002, and 0058-003 do not bind to the CLDN18.1 isoform but selectively bind to the CLDN18.2 antigen.

### Example 4. Intercellular internalization property of selected CLDN18.2-specific monoclonal antibodies

### 4-1. Analysis of cellular internalization using IncuCyte

The cellular internalization property of anti-CLDN18.2 control antibody SC0080 and selected antibodies 0058-001, 0058-002, and 0058-003 was assessed by conjugating each antibody with IncuCyte^{®}FabFluor human red fluorescent reagent (Essen Bioscience, USA), followed by treatment with CLDN18.2-overexpressing HEK293E/18.2 cells or CLDN18.2-negative HEK293E cells. The amount of antibody internalized into the cells was monitored over time. Cells were seeded at 1 × 10⁴ cells per well in a 96-well plate (Nunc, USA) and allowed to adhere for 24 hours. Then, each antibody (4 µg/mL) was mixed with an equal volume of IncuCyte^{®}FabFluor red antibody labeling reagent and incubated at 37°C for 15 minutes in the dark before being applied to the cells. The intracellular uptake amount of the antibody was monitored over a 24-hour period at 15-minute intervals by tracking the accumulation of the antibody in the cells' lysosomes using IncuCyte ZOOM HD/2CLR System (Essen Biosciences, USA) and quantified accordingly.

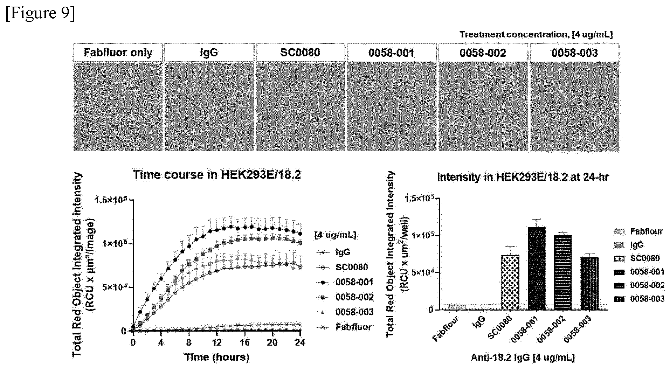

As a result, the selected anti-CLDN18.2 antibodies 0058-001, 0058-002, and 0058-003 that bound to the cell surface did not show intracellular uptake in CLDN18.2-negative HEK293E cells (Figure 8), whereas in HEK293E/18.2 cells, 50% of the antibody was found to be internalized within 5 hours (Figure 9). In particular, selected antibodies 0058-001 and 0058-002 demonstrated superior cellular internalization compared to the control antibody (Y-Biologics, #SC0080; IgG mAb zolbetuximab in-house). These results suggest that all the selected anti-CLDN18.2 antibodies can function as antibodies capable of target-specifically delivering drugs into cells, indicating that they could be developed as antibody-drug conjugates.

### 4-2. Analysis of cellular internalization via immunofluorescence assay

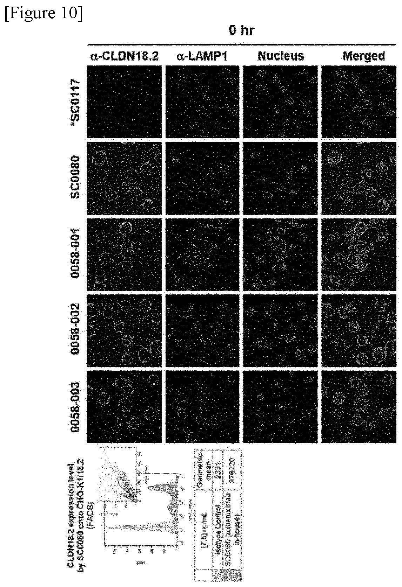

To determine whether the anti-CLDN18.2 antibody can enter the cell after binding to the antigen present on the cell surface, an endocytosis assay was performed in CLDN18.2 overexpressing CHO-K1/18.2 cells, and immunocytochemistry (ICC) was conducted to determine the intracellular location of the antibody. For reference, the CHO-K1/18.2 cells are cells in which CLDN18.2 is overexpressed approximately 161 times compared to CHO-K1 cells, which was determined by performing the same method as Example 2-2 (see the bottom of Figure 10).

In a 24-well plate, 12 mm cover glasses coated with PPL (SIGMA-ALDRICH, #P4707) were placed, and cells were seeded at 5 × 10⁴ cells per well. Each of the anti-CLDN18.2 antibodies 0058-001, 0058-002, and 0058-003 at 10 µg/mL was treated at 4°C for 1 hour to allow the antibodies to bind to the antigens present on the cell surface. Afterward, the cells were washed with cold PBS, and to induce cellular internalization of the antibody, the medium was replaced with pre-warmed complete medium [RPMI-1640 (GIBCO, #A1049101), 1% ANTI-ANTI (Thermo Fisher, #15240062), 10% FBS (Thermo Fisher, #26140-079)], and the cells were cultured for 24 hours at 37°C and 5% CO₂. After culture, the cells were washed three times with PBS to remove the medium, and the cells were fixed with 4% paraformaldehyde. The fixed cells were washed three times with PBS and then reacted at room temperature for 1 hour with an endosome marker antibody diluted in in PBS containing 0.5% saponin (SIGMA, #47036) and 10% FBS. The endosome marker was a late endosome/lysosome marker antibody, and anti-rabbit LAMP1 (Abcam, USA) was used. After marker antibody labeling, the cells were washed three times at 3-minute intervals with PBS containing 10% FBS. Then, secondary antibodies for anti-CLDN18.2 antibody (i.e., FITC-conjugated anti-human IgG, Jackson ImmunoResearch, #709-545-149) and for anti-LAMP1 (i.e., Cy3-conjugated anti-rabbit IgG, Jackson ImmunoResearch, #711-165-152) were diluted 1:200 in PBS containing 0.5% saponin and 10% FBS, and incubated in the dark at room temperature for 1 hour. The fluorescently stained cells were washed three times at 3-minute intervals with PBS and then mounted with DAPI-containing mounting solution (Vector Laboratories, #H1200) on a slide. The cover glass was sealed to prevent air bubbles, and the cells were observed using a confocal laser scanning microscope (ZEISS, LSM9, Germany). The signal information for each fluorescent color is provided in Table 3 below.

**[Table 3]**

| Signal information for fluorescent color | |
|---|---|
| Color | Signal |
| Green | FITC-Antibody |
| Red | Cy3-LAMP1 |
| Blue | DAPI-nucleus |
| Yellow or Orange | Merged antibody and LAMP1 |

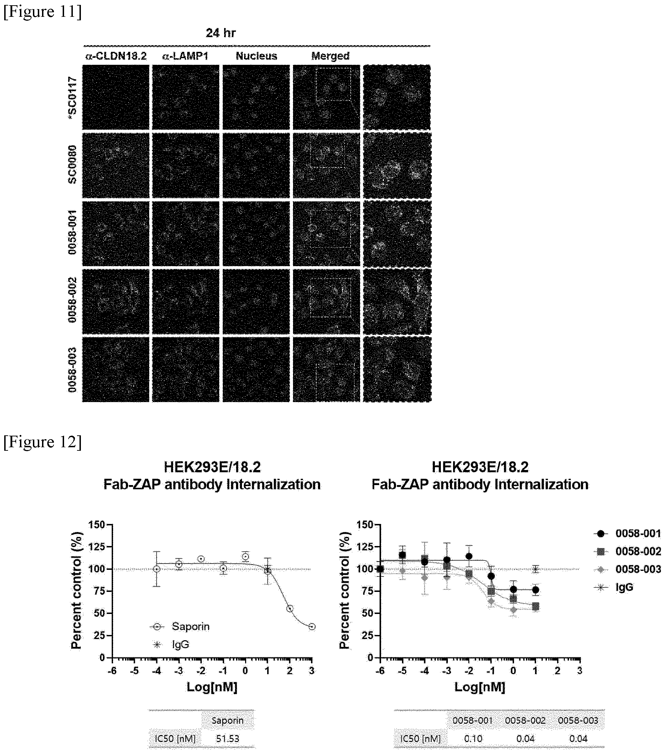

As a result, it was found that the control antibody (SC0080) and the three types of CLDN18.2 monoclonal antibodies entered the cells after inducing endocytosis, while SC0117, an isotype control that does not bind to CLDN18.2, was not observed inside the cells at both 0 hour and 24 hours (Figure 10 and Figure 11). Meanwhile, the anti-CLDN18.2 antibodies that entered the cells showed a high frequency of colocalization with the endosome/lysosome marker antibody anti-LAMP1, indicating that the anti-CLDN18.2 antibodies are delivered to the lysosomes. This indicates again that all the selected anti-CLDN18.2 antibodies 0058-001, 0058-002, and 0058-003 are suitable antibodies for antibody-drug conjugates designed to deliver cytotoxic substances into cells.

### Example 5. Efficacy test of selected CLDN18.2-specific monoclonal antibodies in CLDN18.2-expressing cancer cells

The cellular internalization (antibody internalization) and cell cytotoxicity of the three anti-CLDN18.2 antibodies produced by Y-Biologics Co., Ltd. were determined using the Fab ZAP Human Antibody Internalization Kit (ATSbio, #KIT-51-Z4).

In a biosafety cabinet (#JSCB-1500SB, JSR), HEK293E/CLDN 18.2 overexpressing cell line or HEK293E parental cell line was seeded into each well of a 96-well plate containing 100 µL of the culture medium DMEM (HyClone, #SH30243.01) to a density of 1×10³ cells, and cultured in a 5% CO₂, 37°C incubator for 16 hours. On the day of antibody treatment, the ribosome-inactivating protein saporin (Saporin, ATSbio, #SAP-200) from the Fab-ZAP Kit was prepared by diluting it in the culture medium DMEM from 10,000 nM, which is 10 times the treatment concentration, down to 0.001 nM, creating a total of 8 concentrations. The ZAP spike medium for test antibody dilution was prepared by mixing Fab-ZAP Human (ATSbio, #IT-51-40) in DMEM to a final concentration of 45 nM, and, by using it, the antibody was diluted 10-fold from 100 nM, which is 10 times the treatment concentration, down to 0.00001 nM, creating a total of 8 concentrations. Human IgG (Invitrogen, #31154) was used as the control for the test antibodies. The control material for saporin, Fab IgG-SAP (ATSbio, #IT-67-40), was prepared in DMEM as a cell culture medium to a final concentration of 100 nM, which is 10 times the treatment concentration. Thus-prepared saporin, test antibodies, and Fab IgG-SAP were added to each well of the 96-well plate, which had been cultured for 16 hours, each in 10 µL volumes using a multi-pipette. The plate was then incubated in a 5% CO₂, 37°C incubator for 72 hours.

In a biosafety cabinet, 5.5 mL of DPBS (HyClone, #SH30028.02) and 5.5 mL of XTT from the Fab ZAP Kit (ATSbio, #XTT) were mixed well to prepare 11 mL of DPBS/XTT (1:1) reagent. Then, 92 µL of PMS (ATSbio, #PMS-400) from the kit was added to yield the XTT/PMS reagent. After removing the plate incubated for 72 hours, 50 µL of the XTT/PMS reagent was added to each well that had been treated with the antibody or saporin, and the plate was incubated in a 5% CO₂, 37°C incubator for 2 hours.

After the 2-hour incubation, the plate was removed and the absorbance at wavelength of 450 nm was measured using the GloMax^{®}Discover Microplate Reader (Promega, #GM3000). Cell viability (%) was calculated using the value from the wells without test antibody treatment as 100%, and the IC₅₀ (half maximal inhibitory concentration; the antibody amount required to induce 50% cell death) was calculated using the 4-parameter nonlinear curve (log vs. response-variable slope 4 parameters) in GraphPad Prism (version 9.4).

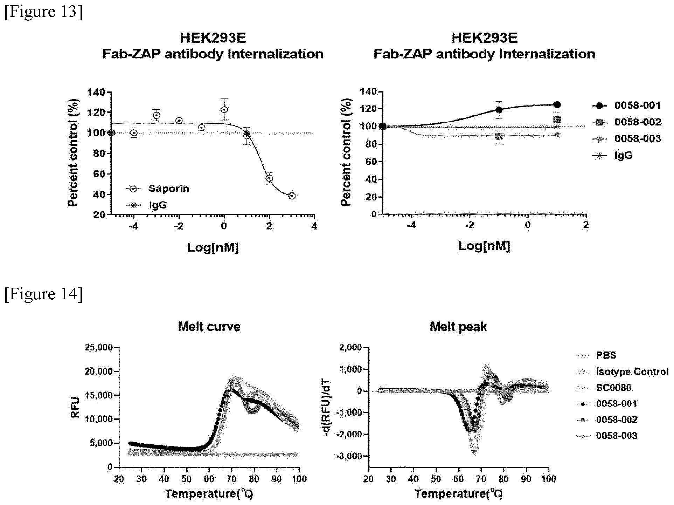

As shown in Table 4, Figure 12, and Figure 13, no cytotoxicity of the test antibodies was observed in HEK293E parental cells without any antigen expression. In the HEK293E/18.2 cell line, in which CLDN18.2 was overexpressed, cytotoxic effects were observed at the highest concentration of the test antibodies (10 nM), and the IC₅₀ values ranged from 0.04 to 0.10 nM. It was accordingly found that the test antibodies are able to induce cell cytotoxicity after antigen-specific internalization, and the effect varies between the test antibodies.

**[Table 4]**

| Cytotoxic effect following cellular internalization | | | |
|---|---|---|---|
| | HEK293E | HEK293E/18.2 | |
| | Cell viability (%) at 10 nM | Cell viability (%) at 10 nM | IC₅₀ (nM) |
| Saporin | 97 | 98 | 51.53 |
| Human IgG | 100 | 100 | N/A |
| 0058-001 | 125 | 76 | 0.10 |
| 0058-002 | 108 | 58 | 0.04 |
| 0058-003 | 91 | 56 | 0.04 |
| N/A: not available | | | |

### Example 6. Thermal stability of selected CLDN18.2-specific monoclonal antibodies

Differential scanning fluorimetry (DSF) was employed to test the thermal stability of the antibodies. The antibody protein was diluted in DPBS to a concentration of 3 µM, and 45 µL was prepared. Then, 5 µL of 200x Sypro orange dye (Thermo, #S6650) was added, and 50 µL of the mixture was dispensed into each qPCR tube (Bio-Rad, #TLS0851-white), and the lid (Bio-Rad, #TCS0803) was closed. qPCR was performed using the Bio-Rad CFX96 real-time PCR system. The qPCR conditions were as follows: a 30-second reaction at 25°C, followed by a gradual increase in temperature by 0.5°C until reaching 99°C, with a 0.5-minute reaction at each temperature. Finally, the reaction was terminated with a 10-second reaction at 25°C. The melting temperature (Tm) was used as the speed constant of antibody unfolding.

**[Table 5]**

| Thermal stability of antibodies | |
|---|---|
| Antibody Type | Tm (°C) |
| SC0080 | 67.00 |
| 0058-001 | 64.50 |
| 0058-002 | 67.00, 81.50 |
| 0058-003 | 67.50, 79.50 |

As shown in Table 5 above, the control antibody and the selected antibodies 0058-002 and 0058-003 had a Tm value above 65°C, indicating good thermal stability, while 0058-001 showed a slightly lower Tm value of 64.5°C. It is believed that the appearance of two Tm values in the 0058-002 and 0058-003 antibodies is due to different rates of unfolding among the domains (CH2, CH3, Fab) that constitute the antibodies, or because the inactivated protein, which unfolded at the first melting temperature, aggregated and then unfolded a second time at the second melting temperature.

## Claims

1. A monoclonal antibody specifically binding to claudin-18.2 (claudin-18 spliced variant 2, CLDN18.2) selected from the group consisting of:
(a) a monoclonal antibody specifically binding to claudin-18.2 comprising a heavy chain variable region containing heavy chain CDR (complementarity-determining region) 1 as described in SEQ ID NO: 1, heavy chain CDR2 as described in SEQ ID NO: 2, and heavy chain CDR3 as described in SEQ ID NO: 3, and a light chain variable region containing light chain CDR1 as described in SEQ ID NO: 5, light chain CDR2 consisting of the amino acid sequence DVS, and light chain CDR3 as described in SEQ ID NO: 6,
(b) a monoclonal antibody specifically binding to claudin-18.2, comprising a heavy chain variable region containing heavy chain CDR1 as described in SEQ ID NO: 1, heavy chain CDR2 as described in SEQ ID NO: 2, and heavy chain CDR3 as described in SEQ ID NO: 8, and a light chain variable region containing light chain CDR1 as described in SEQ ID NO: 10, light chain CDR2 consisting of the amino acid sequence AAS, and light chain CDR3 as described in SEQ ID NO: 11, and
(c) a monoclonal antibody specifically binding to claudin-18.2, comprising a heavy chain variable region containing heavy chain CDR1 as described in SEQ ID NO: 1, heavy chain CDR2 as described in SEQ ID NO: 2, and heavy chain CDR3 as described in SEQ ID NO: 13, and a light chain variable region containing light chain CDR1 as described in SEQ ID NO: 15, light chain CDR2 consisting of the amino acid sequence GAF, and light chain CDR3 as described in SEQ ID NO: 11,
or an antigen-binding fragment thereof.

2. The monoclonal antibody specifically binding to claudin-18.2 or an antigen-binding fragment thereof according to Claim 1, wherein the monoclonal antibody is selected from the group consisting of:
(a) a monoclonal antibody specifically binding to claudin-18.2 comprising a heavy chain variable region described by the amino acid sequence of SEQ ID NO: 4 and a light chain variable region described by the amino acid sequence of SEQ ID NO: 7,
(b) a monoclonal antibody specifically binding to claudin-18.2 comprising a heavy chain variable region described by the amino acid sequence of SEQ ID NO: 9 and a light chain variable region described by the amino acid sequence of SEQ ID NO: 12, and
(c) a monoclonal antibody specifically binding to claudin-18.2 comprising a heavy chain variable region described by the amino acid sequence of SEQ ID NO: 14 and a light chain variable region described by the amino acid sequence of SEQ ID NO: 16.

3. The monoclonal antibody specifically binding to claudin-18.2 or an antigen-binding fragment thereof according to Claim 1 or 2, wherein the monoclonal antibody is selected from the group consisting of a fully human antibody, a humanized antibody, a chimeric antibody, and a recombinant antibody.

4. The monoclonal antibody specifically binding to claudin-18.2 or an antigen-binding fragment thereof according to Claim 1 or 2, wherein the antigen-binding fragment is selected from the group consisting of scFv (single chain variable fragment), dsFv (disulfide stabilized Fv), Fab, F(ab'), and F(ab')₂ that can bind to claudin-18.2.

5. A polynucleotide encoding the heavy chain variable region and the light chain variable region of the monoclonal antibody or an antigen-binding fragment thereof of Claim 1 or 2.

6. A recombinant expression vector comprising the polynucleotide of Claim 5.

7. A non-human transformant transformed with the expression vector of Claim 6.

8. A method for producing a monoclonal antibody specifically binding to claudin-18.2 (claudin-18 spliced variant 2, CLDN18.2) or an antigen-binding fragment thereof, including:
(a) culturing a non-human transformant transformed with a recombinant expression vector comprising a polynucleotide encoding the heavy chain variable region and the light chain variable region of the monoclonal antibody specifically binding to claudin-18.2 or an antigen-binding fragment thereof of Claim 1 or 2 to prepare a culture solution; and
(b) purifying the monoclonal antibody or an antigen-binding fragment thereof from the culture solution of step (a).

9. A pharmaceutical composition for treating cancer comprising the monoclonal antibody or an antigen-binding fragment thereof of Claim 1 or 2 as an active ingredient.

10. The pharmaceutical composition according to Claim 9, wherein the cancer is selected from the group consisting of pancreatic cancer, esophageal cancer, ovarian cancer, lung cancer, gastric cancer, colorectal cancer, liver cancer, biliary tract cancer, gallbladder cancer, breast cancer, kidney cancer, mesothelioma, head and neck cancer, bladder cancer, cervical cancer, endometrial cancer, fallopian tube cancer, gastrointestinal cancer, hematologic cancers, oropharyngeal cancer, melanoma, primary peritoneal cancer, salivary gland cancer, sarcoma, thyroid cancer, glioblastoma, and prostate cancer.

11. The pharmaceutical composition according to Claim 9, wherein the pharmaceutical composition additionally comprises an anti-cancer agent.

12. The pharmaceutical composition according to Claim 11, wherein the anti-cancer agent is a chemotherapeutic agent or an immune checkpoint inhibitor.

13. A method for treating cancer, comprising administering the pharmaceutical composition of Claim 9 to a subject in need thereof.

14. A cancer diagnostic composition comprising the monoclonal antibody or an antigen-binding fragment thereof of Claim 1 or 2.

15. A cancer diagnostic kit comprising the cancer diagnostic composition of Claim 14.

16. An antibody-drug conjugate comprising the monoclonal antibody or an antigen-binding fragment thereof of Claim 1 or 2 conjugated with a drug.

17. The antibody-drug conjugate according to Claim 16, wherein the drug is selected from the group consisting of a cytotoxic agent, an immune modulatory compound, an anti-viral agent, an anti-bacterial agent, an anti-fungal agent, an anti-parasitic agent, a microRNA (miRNA), siRNA, shRNA, a radioactive isotope, and a combination thereof.

18. A chimeric antigen receptor protein comprising
i) the monoclonal antibody of Claim 1 or 2;
ii) a transmembrane domain; and
iii) an intracellular signaling domain **characterized in that** it induces T cell activation upon binding of the antibody of i) to a target antigen.

19. A multi-specific antibody comprising the monoclonal antibody specifically binding to claudin-18.2 (claudin-18 spliced variant 2, CLDN18.2) or an antigen-binding fragment thereof of Claim 1 or 2.
